# EUROPEAN PATENT SPECIFICATION

(11) **EP 1 522 325 B1**
(45) Date of publication and mention of the grant of the patent: **13.06.2007**
(21) Application number: 03022379.6
(22) Date of filing: 06.10.2003
(51) Int. Cl.: A61M 15/00

(54) **Inhaler**
Inhalator
Inhalateur

(43) Date of publication of application: 13.04.2005
(73) Proprietor: Bang & Olufsen Medicom A/S, 7600 Struer (DK)
(72) Inventor: Christrup, Søren, 7600 Struer (DK); Jensen, Søren Dyring, 2500 Valby (DK)
(74) Representative: Tellefsen, Jens J.

(56) References cited:
- WO-A-03/013633
- US-A- 5 769 073
- US-A- 6 012 454

## Description

### FIELD OF THE INVENTION

The present invention relates to an inhalation device for use with a blister pack as well as use of such an inhalator.

### DESCRIPTION OF PRIOR ART

Inhalation devices are known for use with blister packs in which the substance to be dispensed through the inhalation device is held. These devices are generally known as multi-unit-dose devices and can either be reusable devices or disposable devices.

One such device is known from US 5590654 in which a multi-unit-dose device is described where the substances to be dispensed are medicaments which are stored in a blister pack. The blister pack is made from two sheets peelably secured to one another, where single doses of the medicament are stored in separate blister containers provided in the blister pack.

The blister pack is wound into a spiral configuration and placed inside the device. An end part of one of the peelable sheets is attached to a first rotating member and the second sheet of the blister pack is attached to a second rotating member.

In the body of the device a mouthpiece is shaped. A slidable member which can be brought to a first closed position where it shuts off the mouthpiece in relation to the blister pack and a second open position where the blister pack is exposed through the mouthpiece. Once the slidable member has exposed the mouthpiece, a charging member, for example arranged on the slidable member, can be activated. This charging member comprises clutch means and gear wheels, such that when the member is activated by the user manipulating a lever, the two sheets of the blister pack are separated whereby the medicament dose in powder form is exposed in front of the mouthpiece.

When the user inhales through the mouthpiece, means are provided such that an air flow past the medicament container in the blister label will cause the dry powder to be sucked out of the blister pack and suspended in the air current caused by the inhalation of the user.

A special arrangement may be provided between the open blister pack and the outlet of the mouthpiece whereby turbulence is created in the air flow such that powder particles may be de-agglomerated and better distributed and suspended in the inhalation air current.

In a further embodiment of the device according to US 5590645 an extra stage is introduced such that a connection is created between the outlet of the mouthpiece and the blister pack when opening the cover. By further pressing a second button, the peeling mechanism is activated whereby the medicament dose is disposed and through the further step of the user actually inhaling through the mouthpiece, the powder contained in the blister pack will be suspended in the air stream passing over the blister pack and out through the inhalation outlet and thereby into the oral cavity of the inhaler in a similar fashion as described above with respect to the first embodiment.

In comparison to other types of dry power inhalers where the particle size of the powder may be as small as 3-5 µm, the type of inhalers where the doses are packed in blisters have a number of advantages.

In a different type of device as for example illustrated with reference to EP 1067979 A1 a number of doses are stored in one reservoir. This type of device must comprise a dosing unit such that when the user prepares the device for use, a single full dose is measured out in the integrated dosing unit. These types of devices usually comprise a very large number of doses. Furthermore, a number of the known devices need to be operated in a certain manner, namely that the longitudinal axis through the inhalation mouthpiece must be vertical during the measuring out of the dose in order for this task to be carried out in a reliable way. The consequence of not keeping the device vertical is that only part of the full dose or nothing at all becomes available to the user during the preparation stage. As the doses usually are very small, i.e. a few milligrams, it is impossible for the user to judge whether a full dose (or nothing at all) was dispensed.

Also, with respect to the dose counter, this type of dispenser/inhalation device has a number of drawbacks. When the device is manipulated in order to prepare for the dispensation of a dose, the dose counter is advanced one click. The user will then know how many doses are left or have already been dispensed. However, in cases where the device is not maintained in a vertical position during measuring out of a dose such that only part of a dose is actually made available to the user, the dose counter counts a full dose. In cases where the medicament is expensive, the waste due to counted, but not dispensed, doses may be substantial.

A further problem with this type of device is the fact that when opening and closing the device, for example in connection with measuring out a dose, it is possible for moisture from the ambient air to gain access to the reservoir in which the medicament is stored in powder form. With powder particles being as small as described above, it may cause agglomeration due to water absorption on such a scale that it will not be possible to dispense the desired amount of powder through the measuring unit or the powder inside the reservoir may agglomerate to such an extend that it will not be possible to dispense all the doses contained in the reservoir. A further problem is the fact that fine powder will have a tendency to swell when exposed to moisture. This can cause agglomerates or larger particles to be dispensed by the device. As it is desirable to inhale fine powder in order to assure that the powder is transported to the lungs, the formation of larger particles may result in the medicament dose not being suspended in the air stream, but rather being deposited in the oral cavity, whereby that particular medication, for example asthma medication, will have no effect on the user. The uniform dosing which is desirable may for one or more reasons as discussed above not be attainable with this type of devices.

From WO 03/013633 is a further device known, wherein the medication is packaged in separate compartments in a blister, and that the blister is biased such that when a user activates the device and sucks in the mouthpiece the medication is released into the air stream created by a user. WO 03/013633 disclose all features of the preamble of claim 1 and is considered as the closest prior art for the present invention.

It is an object of the present invention to provide an inhalation device of the type wherein the substance to be inhaled, whether it is a medicament or the like, is stored inside the device in a blister pack. A further object of the invention is to make sure that once a dose is exposed through the mouthpiece, it will be inhaled by the user. This is contrary to the device mentioned above disclosed in US 5590645, where the dose may be exposed without the user actually inhaling the dose, as the dose may accidentally be emptied out either inside the device or out through the mouthpiece if, for example, a period of time occurs between the time of exposure of the dose due to the peeling action inside the device and before the actual inhalation by a user.

### BRIEF SUMMARY OF THE INVENTION

According to the present invention an inhalation device is provided for use with a blister pack, where said blister pack comprises a base foil and a lid foil peelably secured to each other, where said blister pack has a plurality of containers formed in either the base foil or the lid foil, for containing a substance, wherein the containers are spaced along the length of the blister pack,
wherein the device comprises :
- an outer shell wherein in a first end of the outer shell a mouth piece is provided;
- an inner winder arranged coaxially inside said outer shell, with means for receiving and holding one end of one of the foils making up the blister pack;
- a breath activated trigger mechanism comprising biasing means for rotating the inner winder a preset distance when said trigger mechanism is triggered.

In this manner it is assured that by having a breath activated trigger, i.e. a trigger which is only activated by the inhalation of a user, the dose will only become exposed due to inhalation and thereby due to an air current from the inhalation triggering the exposure and dispensation of a dose into the oral cavity of a user.

As described above the packaging of substances such as medicaments or other dry powder material in blister packs comprises a number of advantages. Firstly, it is under factory conditions possible to measure out the desired dose very exactly and place it safely in a blister pack such that the size of the dose can be determined very accurately. Furthermore, by choosing suitable blister pack materials, i.e. the materials making up the base foil and the lid foil as well as the adhesive securing the two foils to each other, a moisture insensitive package may be produced. The method of securing the two foils to each other may be chosen among a number of suitable solutions, but especially preferred is the use of adhesives chosen according to the materials from which the blister pack is made, or heat fusion of the two foils or weak welding of the two foil materials.

Depending of the method of assembling the two blister foils in order to address both the requirements relating to moisture resistance and also creating an adhesion between the two foils strong enough to store and handle the substances stored in each blister label without these being damaged may be optimised according to circumstances. It is naturally advantageous to minimise the area of the blister label set aside to securing the two foils together in that this leaves more space for further doses on the blister pack and thereby inside the device as such or provides for a small device.

The trigger mechanism will when activated by a user's breath release the inner winder which is biased for rotation such that one of the foils making up the blister pack will be wound onto the inner winder and thereby exposing the substance stored in the blister pack. As this is only exposed during the inhalation, it is assured that the user has placed the mouthpiece in the mouth. The exposed substance will, due to the air flow through the device and past the container in the blister pack, be transported into the oral cavity of the user.

In comparison to the prior art devices as mentioned above where the doses or substances are exposed prior to inhalation, the new and inventive device according to the present invention only exposes the dose during the inhalation, whereby it is assured that the substance will be transported with the air flow of the inhalation into the oral cavity of the user.

The trigger mechanism is made for rotation due to the biasing means such that it will rotate a distance corresponding to one container on the blister pack after which a limiting member will engage and prevent further rotation until next time the trigger mechanism is activated.

In a further advantageous embodiment the outer shell has a cylindrical configuration, optionally substantially circular, elliptical or oval in cross-section, and that on the inner side of the outer shell grooves are provided in the longitudinal direction substantially corresponding in size to the blister containers, said grooves extending substantially the length of the outer shell. By providing grooves corresponding to the size of the blister containers, the blister pack may be placed and held in a secure manner inside the device such that the process of winding the foils of the blister pack and thereby exposing the substances may be done in a reliable way. Furthermore, the blister pack will not rotate due to the rotation of the inner winder as the grooves provide resistance against this rotational movement. The grooves also provide for longitudinal guidance for the entire blister pack in relation to the outer shell of the device.

In a further advantageous embodiment a trigger mechanism is provided, which trigger mechanism comprises:
- one or more wings provided integral with the circumference of the inner winder;
- a biasing member coaxially arranged inside the inner winder urging a substantially constant rotational biasing force on the inner winder;
- a central axle with a cross-section comprising a number of knobs arranged in one end of said axle;
- a flap pivotable about an axis perpendicular to the longitudinal axis of the device comprising at least one knob being arranged for releasable engagement with a wing of the inner winder, said flap being arranged adjacent the opposite end of the mouthpiece, and the flap having an area and shape substantially corresponding to the inner cross-section of the outer shell;
- an activating member optionally operatively connected to a mouthpiece cap or an activating button said activating member comprising an engagement section, said section being arranged for releasable engagement with a wing of the inner winder.

By this construction it is assured that once the device is purposely activated by manipulating the activating member such that a first wing is released, the apparatus is ready for use and the user might place it in the mouth. Thereafter, by inhaling air through the device, the flap which is pivotally arranged inside the device on an axis perpendicular to the longitudinal direction will tilt. Due to this tilting movement, a knob which engages a further wing on the inner winder will come out of engagement and thereby allow the inner winder to rotate and thus peel off the foil from the blister pack and expose the substance of the blister pack container.

The provision of a substantially constant rotating biasing force, for example in the shape of a spring, facilitates the rotational movement of the inner winder, substantially instantaneous once the device is activated, whereby it is assured that the substance in the blister pack container is exposed at a very early stage during the inhalation by the user such that it must be presumed that sufficient air will pass by the open container in the blister pack in order for the substance to become suspended in the air stream passing by the exposed blister container.

In a further advantageous embodiment the inhalation device further comprises a blister cover having a longitudinal slit, said blister cover being arranged coaxially inside said outer shell at a distance allowing for the accommodation of a helically wound blister pack, and outside said inner winder.

By providing a blister cover it is possible to ensure that the angle between the blister pack and the inner winder is constant. By being able to keep this angle constant the force necessary to peel the lid foil and the base foil apart may also be considered substantially constant. In this manner, it is possible to optimise the distance between two containers on the blister pack in that the force needed to pull two foils apart may be determined very accurately and at the same time the moisture penetration rate between the two foils can also be calculated.

In order to store as many blister pack containers and thereby doses in the device, the blister pack can advantageously be stored wound up in a helix such that the entire blister pack will be shaped as a cylinder, where each container is in the same longitudinal line as the adjacent container corresponding to the grooves provided in the outer shell. When assembling the device a number of superposed containers arranged in the same line will fit inside a longitudinal groove in the outer shell such that firm storage of the blister pack in relation to the device may be achieved. It is evident that minimising the areas on the blister pack used for peelably securing the foil together result in the fact that more doses can be stored inside the device.
In practice it has been shown that sixty containers arranged in a blister pack as disclosed above provides a good compromise between the size of the device and the provision of a multi-dose device.

It should, however, within the framework of the present invention be construed that any number of containers in the blister pack may be contemplated. In this connection, and especially with the helical configuration, it is possible to have two, three or more layers of helically wound blister labels arranged one inside the other in that by this further provision of the blister cover a uniform peeling action can be achieved.

In connection with the peeling apart of the two foils making up the blister pack, the outermost edge, where the two foils are still connected, is called the peeling front. It is important that when the inner winder is rotated the peeling front moves across a blister container and stops in the area between two blister containers such that the substances stored in the blister containers will not be exposed after inhalation. This may be controlled by the arrangement of the wings on the inner winder in relation to the position of the wings to the knobs and notches of the activating member and the flap.

According to the number of wings or number of knobs and notches, the angle through which the inner winder will have to travel in order to uncover a blister container may be controller.

For some substances it is necessary in order to achieve the desired effect of the treatment in cases where it is a medicament that the blister containers have a certain size whereby the inner winder needs to rotate through a substantial angle.

For other substances, which also may be medicaments, a very small dose is required. In these instances the distance between two blister containers may be relatively small such that only a small angular travel by the inner winder is required. Again, this can be assured by the provision of more or less wings on the inner winder in relation to the knobs and notches provided on the activating member and the flap.

Although it is contemplated to be able to use the same device with different types of substances such that the rotation of the inner winder may be set at a relatively large angle in order to be able to peel off the foil of any size of container, especially in embodiments where the device is made for multiple uses, the grooves inside the device should be given the maximum size of the blister containers. This embodiment, however, requires that the blister label is adapted such that the peel front always will be placed between two containers when the device is not triggered. The area between two containers must therefore have a corresponding length. For the purpose-build devices and especially for the single use devices, it is possible to optimise the number of containers arranged on the blister pack and the angular rotation of the inner winder such that the peeling front may be assured to be arranged between two containers when the device is not activated.

In a further advantageous embodiment a hinged cap for covering the mouthpiece is provided, and the cap is connected to the activating member, such that pivoting of the cap engages or disengages the engagement section. By providing a cap it may be assured that foreign matter, dirt and the like does not enter the device or become stuck in the mouth piece as such. By connecting the hinge cap to the activating member is achieved that when the cap is moved from its closed to its open position the activating member is activated and thereby the trigger mechanism is activated. There is a relationship for-the user between physically making the device ready for use by removing/pivoting the cap and the device itself preparing via the trigger mechanism to expose a dose. Furthermore, opening and closing of the cap member may be carried out without exposing a dose whereby a user repeatedly opening and closing the cap, maybe absentminded, will not expose doses which might otherwise be wasted by this opening and closing process. Only by actually inhaling and thereby triggering the trigger mechanism the substance will be exposed at the same time as an air flow is generated through the device.

In a further embodiment of the invention an activating button is provided in the outer shell, either as a sliding button or as a depression button, where said button is connected to the activating member, such that activation of the button engages or disengages the engagement section.
In this configuration the device also, as was the case with the pivoting of the cap, requires a positive action in order to prepare the device for use. And again, as was the case for the hinged cap, a user's play with the button does not release or expose a substance kept in the blister pack, but only prepares the device for action. Thereby, the same advantages as mentioned above, namely that the substance is only exposed due to the actual creation of an air stream through the device due to inhalation by the user, may be achieved. In the prior art the dose may be exposed prior to creating the air stream, whereby it is riot safeguarded that the dose has not disappeared before the air stream by inhalation is created.

In a further advantages embodiment, the device is suitable for substances which may be chosen among fine or coarse powders such as medicaments, candy, or solids such as pills, capsules, candy or liquids such as medicaments, mouth wash, candy and the like.

Although it is acknowledged that the majority of uses for this type of devices are for the dispensation of medicaments which are to be taken orally, the present invention also makes it possible, due to its simple and reliable construction, to dispense other substances, whether it be fine or coarse powders or solids such as pills, capsules or the like, or even liquids. In embodiments where it is desirable to dispense powders-or-liquids, the mouthpiece may advantageously be equipped with means whereby a turbulent air current is created such that the powder or liquid becomes better and more evenly suspended in the created air stream.

Furthermore, for some uses where patients for one reason or another need to take medication in pill or capsule form, this may advantageously be provided in the inventive device as described with the present invention. If the medication in pill or capsule form is provided in the traditional glass or blister pack, it is not assured that once a user takes a pill out of the glass or squeezes a pill out of the blister pack that this pill will actually be placed in the mouth. The user may be distracted, may loose the pill or otherwise arrive in a situation where the pill either has disappeared or where the user has forgotten that a pill was taken out of the package. Especially in cases where either the medicament is very expensive or very critical to the health of the user, this constitutes an undesirable situation. Furthermore, users suffering from memory defects may believe that the medicament once dispensed has also been taken. It is, therefore, in these and other circumstances advantageous that the substance which is to be dispensed, whether it be a capsule or a pill containing a medicament or the like, will only be dispensed due to an active inhalation by the user, whereby it is assured that the pill or capsule arrives in the mouth of the user. Of course, it cannot be guaranteed that the user will also swallow the pill or capsule once it is in the oral cavity, but the disadvantages mentioned above may all be alleviated by this manner of dispensing a dose.

In cases where the substance is for example mouth wash, it may be desirable for the user to have the device in a pocket, bag or the like, such that a dose may be dispensed discretely. Furthermore, by optimising the blister pack the substance may be kept fresh, whereas these types of substances kept in bottles or the like may have a tendency to deteriorate over time due to the increasing exposure to the ambient air.

In some cases it may be advantageous to provide medication in powder form which normally is to be taken in pill or capsule form. For asthma medication the grain size of the powder must be very fine such that the powder effectively will be carried by the air stream caused by inhalation into the airways and lungs. For other medicaments where the medicine has to be assimilated by the intestines, it is desirable to have a coarser powder such that the medication enters the oesophagus. In some instances it has been a problem that the intestines of a patient could not or only very poorly decompose a pill or capsule. This may be due to the support or carrier agents which are necessary for the production of pills or capsules. By providing the medication in powder form, the need for support or carrier agents such as gelatine or other substances is avoided, and the surface of the medication grains is much larger than that of a pill or capsule such that the ability of the body to assimilate the medication is greatly increased. This may also apply to the time factor such that the medication becomes active faster.

With the present device a device is thus provided which is simpler to use than prior art devices. The use of the device is self-explanatory; remove the cap and inhale. Furthermore, the actual dosing of substance is insensitive to orientation of the device and due to the use of blister packs the problems relating to moisture are avoided. As the dose counter is only activated as a function of actually dispensing a dose, a reliable and exact dose counting mechanism is achieved, which may furthermore comprise a warning when only a few doses are left in the device.

Traditionally, the doctor prescribing for example asthma medication needs to spend time instructing the patient on how to use the device. With the present device a very intuitive and straight-forward mode of operation is provided, whereby the need for a doctor to give instructions is minimised and, at the same time, due to the straight-forward mode of operation, the use may feel safe and secure using the device.

An embodiment of the present invention will be explained in more detail with respect to the accompanying non-limiting drawing, in which
- fig. 1: illustrates an exploded view of an embodiment of the invention,
- fig. 2: illustrates a cross-section of an embodiment of the invention,
- fig. 3: illustrates an exploded view of a further embodiment of the invention,
- fig. 4: illustrates a closed, respectively open, device according to the invention,
- fig. 5a-d: illustrate the sequence of releasing a substance,
- fig. 6: illustrates a dose counting mechanism,
- fig. 7: illustrates an alternative dose counting mechanism and
- fig. 8: illustrates a blister pack.

The same or corresponding features will be denoted with the same reference numbers throughout the description.

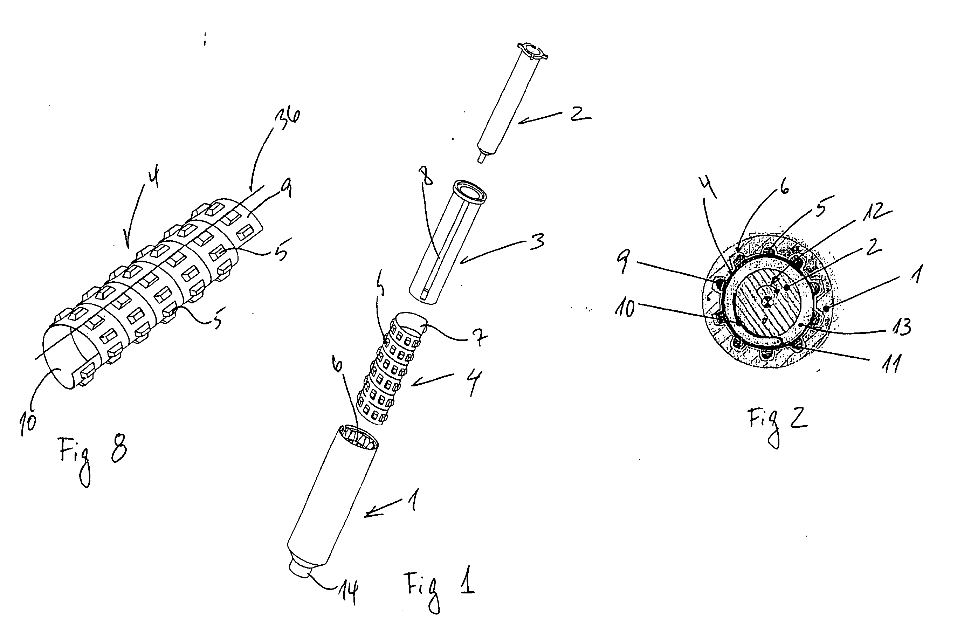

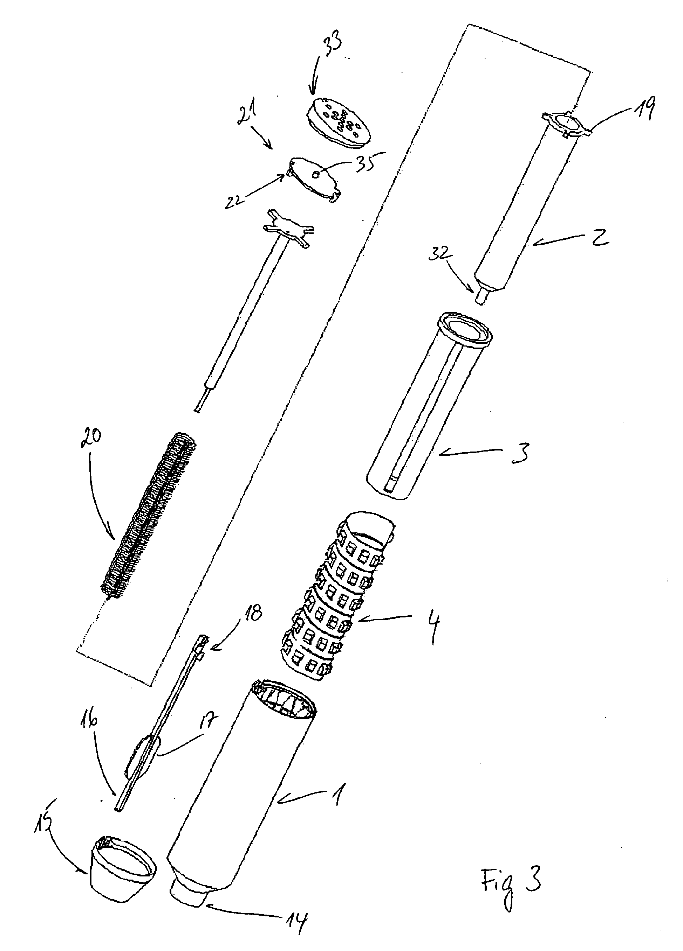

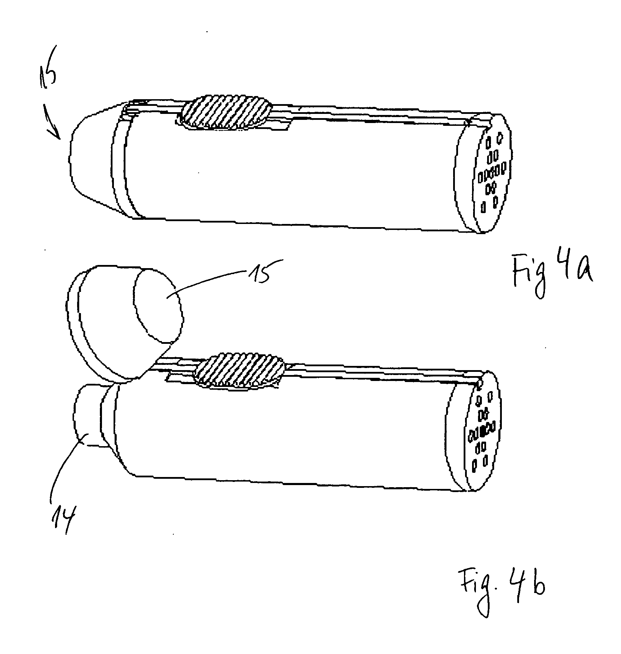

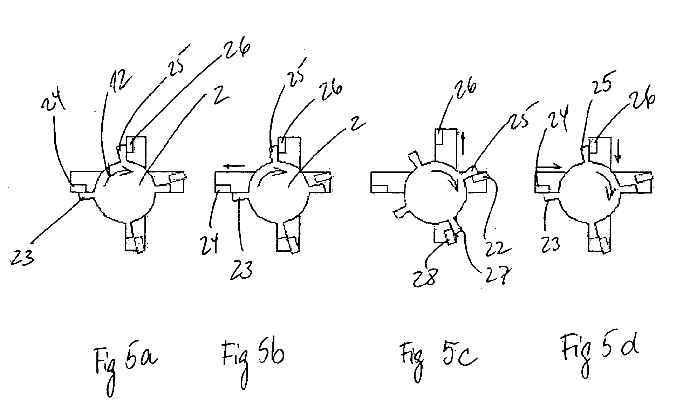

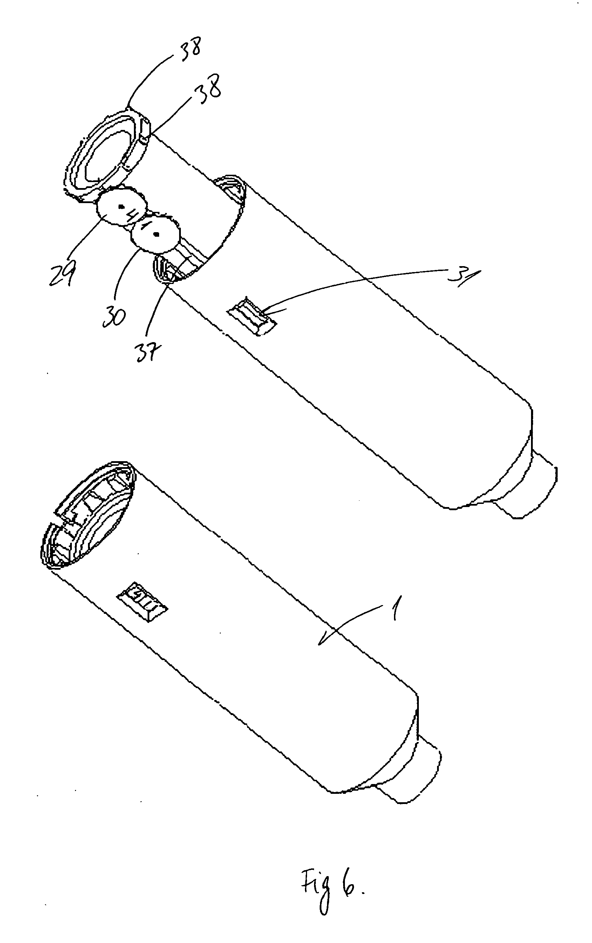

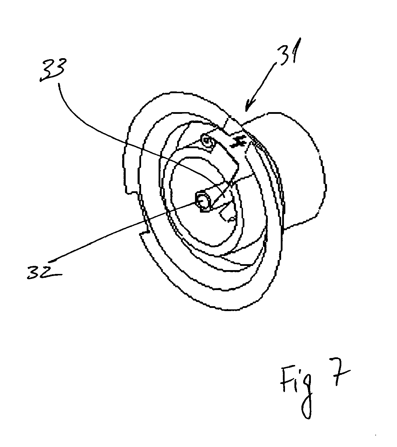

In fig. 1 is illustrated an exploded view of a device according to an embodiment of the invention. The device comprises an outer shell 1 and an inner winder 2. In some embodiments it might be advantageous additionally to provide a blister cover 3. Furthermore, a blister pack 4 for use with an embodiment of the invention is illustrated.

The device is assembled by arranging the blister pack 4 coaxially inside the outer shell 1. Inside the outer shell 1 grooves 6 may be provided arranged longitudinally in the outer shell 1. The size of the grooves corresponds to the size of the containers 5 in blister pack 4. By winding the blister pack 4 it will be achieved that the individual containers 5 in its winding will be superposed such that a number of containers 5 in a blister pack will fit inside and be held by the grooves 6 provided in the outer shell 1.

Where a blister cover 3 is provided, this is arranged coaxially inside the blister pack 4 and an end 7 of the blister pack 4 is threaded through a slit 8 provided longitudinally in the blister cover 3.

Turning shortly to fig. 8, a blister pack 4, which may be used with the device according to the present invention, is constructed by two foils 9, 10. In the base foil 9 a number of blister containers 5 are provided. A substance may thereafter be placed in the container 5, after which the lid foil 10 is secured to the base foil 9 such that the lid foil may be peeled from the base foil 9 and thereby exposing the substances stored in the containers 5.

Turning back to fig. 1, one of the foils 9, 10 of the blister pack 4 is attached to the inner winder 2. By rotating the inner winder 2, one of the foils 9, 10 will be wound around the inner winder 2 and thereby open one or more of the containers 5 and thus expose the substance stored in said container.

This aspect is illustrated in fig. 2 which is a cross-section through a device according to the invention.

In this embodiment the lid foil 10 has been secured to the inner winder 2 such that by rotating the inner winder in a direction indicated by the arrow 12, the lid foil will be peeled off the base foil 9 and thereby exposing a substance placed in a container 5.

The point where the lid foil is just peeled off the base foil is called the peeling front 11. As the lid foil is peeled off the blister pack 4, the lid foil 10 will be helically wound onto the inner winder 2 corresponding to the helically wound blister pack 4.

In one end of the outer shell 1 a mouthpiece 14 (see fig. 1) is provided. As the user places the mouthpiece in the mouth in order to inhale a dose of whatever substance is stored in the blister pack containers 5, a air stream will be created in the space 13, see fig. 2. As this air stream passes the exposed substance in the container 5, this substance will be blended into the air stream, let through the device and out through the mouthpiece 14 and into the oral cavity of a user.

Especially where the substance in the containers 5 are medicaments, it is important that the proper dose is administered. This in turn requires that once the inner winder 2 is brought to rotate, illustrated by the arrow 12, only one container 5 is exposed. The rotation 12 shall therefore move the peeling front a very well-defined and predetermined distance, namely exactly the distance from the area where the base foil and the lid foil 9, 10 are secured to each other, past a container 5 and stop the peeling at the following place where the lid foil and base foil are secured together. For this purpose a trigger mechanism is provided.

This trigger mechanism is illustrated and will be explained with reference to fig. 3. The device is assembled as explained with reference to fig. 1. Additionally, a cap 15 is provided. The cap 15 is fastened to the outer shell 1 by appropriate hinge means (not illustrated). The cap 15 is, furthermore, connected to an activating member 16.

The activating member may be provided with means 17 for manual manipulation such that sliding the means 17 in the longitudinal direction of the outer shell 1 will cause the cap 15 to pivot and thereby expose the mouthpiece 14. In the opposite end of where the cap 15 is fastened to the activating member 15, an engagement section 18 is provided.

Furthermore, at the upper end of the inner winder 2 a number of wings 19 are provided. Coaxially inside the inner winder 2 a biasing means 20, for example a helical spring or any other means which may bias the winder to rotate as illustrated with reference to fig. 2.

The trigger mechanism also comprises a flap member 21. On the flap 21 is provided at least one knob 22 and the flap is designed to be pivotally arranged along an axis perpendicular to the longitudinal axis of the inhalation device.
In order to explain in detail the functioning of the trigger mechanism and the cooperation, especially with the inner winder and thereby the exposure of the substances kept in the containers 5 of the blister pack 4, reference is now made to fig. 5a-d. Fig. 5 illustrates the cooperation between the wings 19 of the inner winder 2, the engagement section 18, the activating member 16 and the knob 22 provided on the flap 21.

When the device is closed, as illustrated in fig. 4a, where the cap 15 covers the mouthpiece, the corresponding position of the trigger mechanism is illustrated with reference to fig. 5a.

A first wing 23 is, due to the biasing force of the spring member 20 in the direction of rotation 12, hindered in rotation by a first notch 24 provided on the engagement section 18 of the activating member 16. Furthermore, a second wing 25 is engaging a first knob 26 on the pivotally arranged flap member 21.

As the device is opened, as illustrated in fig. 4b, where the cap 15 has been pivoted in order to expose the mouthpiece 14, the trigger mechanism will be in the position illustrated with reference to fig. 5b.

The pivoting of the cap 15 moves the engagement section 18 such that the first notch 24 is released from the engagement with the first wing 23. The inner winder 2 is still refrained from rotation due to the engagement between the second wing 25 and the first knob 26 of the flap.

When a user inhales through the device by placing the mouthpiece 14 in the mouth and creating an air stream in the space 13, the flap 21 will pivot whereby the knob 26 will disengage its engagement with the second wing 25 as illustrated in fig. 5c. Hereby the inner winder will rotate and peel off part of the lid foil, i.e. the peeling front 11 will move a predetermined distance corresponding to the second wing 25 moving from engagement with the knob 26 to engagement with a second knob 22 provided on the engagement section 18. This distance corresponds to the peeling front moving from one area where the base and lid foils are secured together to the following area where the foils are secured together, exposing exactly one container and thereby one dose of the substance stored in the container 5.

The rotation of the inner winder 2 happens substantially instantaneously in that the air stream created by the inhalation of the user pivots the flap, whereby the substance in the blister container 5 is exposed at a very early stage during the inhalation process. The user's inhalation thereby triggers the movement of the flap, whereby the peeling action is initiated such that the substance will gain access to the air stream created in the space 13 and thereafter by means of the air stream led into the oral cavity of the user through the mouthpiece 14.

The rotational movement of the inner winder 2 is haltered by the engagement of the second wing 25 with the second notch 22 of the engagement section as well as a third wing member's 27 engagement with a second knob 28 provided on the flap member (see fig. 5c).

When the rotational and sliding movements as explained with reference to fig. 5c are completed, the inhalation process and thereby the dispensing of a dose is concluded. The user will, in order to prepare the device for another dispensation of a dose, have to close the cap back into its closed position as illustrated in fig. 4a. By this movement the activating member and thereby the engagement section will move back into their original positions as illustrated in fig. 5d. At the same time the flap will be urged back into its original starting position such a "new first wing" 23 will engage the first notch 24 and a "new second wing" 25 will engage the first knob 26 on the flap.

Hereafter, the device is made ready for an additional dispensation which is initiated by opening the cap 15, whereby the sliding and rotational movements as described with reference to fig. 5a and 5b will occur. Subsequent inhalation triggers the movements as explained with reference to fig. 5c and resetting of the device as well as closing of the device as illustrated with reference to fig. 4a is carried out by the movements as illustrated in fig. 5b.

In the description of the device with reference to fig. 1 and fig. 3, the blister cover 3 was mentioned. The blister cover serves to ensure that the peeling angle with which the base foil is separated/peeled from the lid foil always remains substantially constant such that the force needed in order to peel and thereby expose the substances kept in the containers 5 can remain substantially constant. Furthermore, the blister cover may be designed such that once the peel front 11 has passed the container and thereby exposed the substance kept in this, the exposed container will be superposed with the slit 8 in the blister cover such that the air stream in the space 13 will concentrate on evacuating the container 5 whereby the contents may be suspended in the air stream and via the mouthpiece be brought into the oral cavity of the user.

When the device is used for dispensing medicaments such as for example dry powdered asthma medication, it is desirable to be able to indicate either how many doses have been dispensed or have many doses there still is left on the device. For this purpose, a dose counter may be provided in the device. In fig. 6 and 7 two different alternative dose counters are illustrated. It should, however, be evident that other types of dose counters than those illustrated may be used with the present device.

In fig. 6 a dose counting mechanism is illustrated comprising two discs 29, 30. The discs may be arranged on for example an independent member 37 arranged inside the outer shell. On the inner winder or the blister cover notches may be provided such that as the inner winder is caused to rotate as explained above, one of the notches 38 will engage means on the disc 29 and cause this to rotate and thereby count a dose dispensed. On the disc 30 numbers indicating 0 to 9 are marked and after completing one revolution, the following count will trigger the second wheel 29 where the ten group is indicated.

In order to indicate to the user how many doses there either are left or have been dispensed, a window 31 is provided in the outer shell. Depending on how the counting mechanism, i.e. the notches 38, causes the two discs 29, 30 to rotate, the counter can be made to count upwards or backwards as desired.

The triggering of the dose counting mechanism may also be carried out by the knob 22 provided on the flap 21. It is advantageous to couple the triggering of the two dose indicating wheels 29, 30 to the movement of the flap or as explained above to the rotational movement. In addition to triggering the dose counting mechanism, the flap also initiates the peeling and thereby the exposure of the substance stored in the blister pack container 5. In this manner, there is a secure relationship between the dispensation of the dose and the counting of that dose.

With reference to fig. 7, an alternative dose counting mechanism is illustrated. A tape 33 is fastened to a disc end 32 of the inner winder. On the tape is printed the numbers 1 through the number of doses contained in the blister pack, either in ascending or descending order, depending on the dose counting method. Adjacent the mouthpiece a window 31 is provided through which the number printed on the tape 33 may be read. In this example the number "4" may be read through the window 31.

As the inner winder rotates due to the dispensation of doses from the containers of the blister pack as explained with reference to figs. 5a through 5d, the tape 33 will be wound onto the distal end 32 of the inner winder 2. In addition to the numbers, the tape may be given a colour or different colours such that as long as a certain number of doses remain unexposed in the blister pack, the tape is green and when only a critical number of doses remain in the blister pack, the tape may turn red. The colour coding can be any suitable colour coding and the tape may be provided with numbers in addition to the colour coding, numbers alone or colour coding alone.

Colour coding is a known way of indicating what kind of medication is provided in the device. Therefore, the device according to the present invention may, in a corresponding manner, be supplied with or produced in a material having the colour code corresponding to the substances stored in the containers 5 of the blister pack 4. The colouring of the device may also consist in only the cap member 15 is coloured in order to indicate the substances maintained in the device.

In order to maintain the coaxially arranged elements of the device as well as the flap inside the outer shell, a ventilation plug 33 may be provided. In the ventilation plug a number of holes are provided such that when the user inhales through the mouthpiece 14, the ventilation plug will let in sufficient air through these holes in order to create an air stream in the space 13, which is sufficient in order to suspend the substances stored in the containers 5 of the blister pack in that air stream.

If desired, a coloured notch 35 may be provided on the side of the flap facing the ventilation plug. This notch must be superposed with one of the holes provided in the ventilation plug 33. When the flap 21 pivots, the notch 35 will be visible in the ventilation plug 33 and thereby indicate to the user that the device is ready for use. As the inhalation has been carried out and thus as the peel front has passed and exposed a container 5 in the blister label and thereby dispensed a dose, the flap will pivot and thereby the coloured notch will not be visible in the ventilation plug. This is an indication to the user that the dispensation of a dose has been successfully carried out.

With reference to fig. 8 is illustrated a blister pack which is suitable for use in a device as explained above. The blister pack is one long strip where the number of blister containers 5 are provided in a long row.

The blister pack is, as explained above, made from two foils, a lid foil 10 and a base foil 9 in which base foil 9 the blister containers are formed. After forming the containers, a substance such as a dry powder, medicament, a liquid, pill or capsule may be placed in the container, whereafter the base foil 10 is secured to the lid foil. The securing of the two foils should be done in such a way that it is assured that ingress of moisture which can be detrimental to the substances stored in the containers, especially if these substances are in the shape of dry powders or where the ingress of moisture will decay the substances in the blister pack, is avoided. On the other hand, the two foils should not be secured together in such a way that it is not possible to peel one foil from the other in order to expose the substances stored in the containers 5.

When designing the blister foil, the containers shall be spaced such that when the blister pack is wound into a helical configuration as illustrated in fig. 8 containers 5 in different windings will be arranged along the same longitudinal line as indicated by the line 36. Also, the blisters 5 may be arranged at an angle with respect to the longitudinal direction of the blister pack 4 in order to compensate for the helical winding of the blister pack, such that where the containers have an elongated configuration as illustrated in fig. 8, the longitudinal axis of this elongated configuration will be angled such that the longitudinal direction of the containers corresponds to the longitudinal direction of the helically wound blister pack and thereby the longitudinal direction of the grooves 6 arranged in the outer shell 1 of the device.

The device is generally suitable for inhalation of any type of substance which can be package in a blister pack as described above. Traditionally, these devices are used for providing medication in dry powder form, but also liquids, whether or not it is medication, mouthwash or the like, or solids, for example pills, capsules, candy or the like, may be dispensed with the device according to the invention.

The device provides a simple and yet safe manner of dispensing the substance and assuring that the substance is only dispensed once the user intends to inhale the substance and thereby has placed the mouthpiece in the mouth. It is only by creating an air stream through the device that the exposure of the substance occurs and, therefore, the transport of the substance into the oral cavity is assured. Thus, the user will be assured that even though the cap or the button may be activated without the intention of dispensing a dose, no actual dispensation occurs and, therefore, the waste is minimised. Also, when not dispensing doses in vain, it is assured that the actual amount of doses stored in the blister pack containers 5 remains safe. A further advantage with the present device is that the dispensation of a dose is not dependent of the direction into which the device is held during the dispensation/inhalation. Therefore, bedridden patients may take their medication lying down, standing up or in any other desired position.

## Claims

1. An inhalation device_for use with a blister pack (4), where said blister pack (4) comprise a base foil (9) and a lid foil (10) peelably secured to each other, where said blister pack (4) has a plurality of containers (5) formed in either the base foil (9) or the lid foil (10), for containing a substance, wherein the containers (5) are spaced along the length of the blister pack (4), the inhalation device comprising
- an outer shell (1) wherein in a first end of the outer shell a mouth piece(14) is provided, and
- an inner winder (2) with means for receiving and holding one end of one of the foils (9,10) making up the blister pack (4);
**characterised in that**:
- the inner winder (2) is arranged coaxially inside said outer shell (1), and the device further comprises
- a breath activated trigger mechanism comprising biasing means for rotating the inner winder (2) a preset distance when said trigger mechanism is triggered, such that the one of the foils making up the blister pack will be wound onto the inner winder thereby exposing the substance stored in the blister pack.

2. Inhalation device according to claim 1, wherein the outer shell (1) has a cylindrical configuration, optionally substantially circular, elliptical or oval in cross-section, and that on the inner side of the outer shell (1) grooves (6) are provided in the longitudinal direction substantially corresponding in size to the blister containers (5), said grooves (6) extending substantially the length of the outer shell (1).

3. Inhalation device according to claim 1, wherein the trigger mechanism comprises :
- one or more wings (19,23,25) provided integral with the circumference of the inner winder (2);
- a biasing member (20) coaxially arranged inside the inner winder (2) urging a substantially constant rotational biasing force on the inner winder (2);
- a central axle with a cross-section comprising a number of knobs (22,26) arranged in one end of said axle;
- a flap (21) pivotable about an axis perpendicular to the longitudinal axis of the device comprising at least one knob being arranged for releasable engagement with a wing (13,23,25) of the inner-winder (2); said flap being arranged adjacent-the opposite end of the mouthpiece(14), and the flap(21) having an area and shape substantially corresponding to the inner cross-section of the outer shell (1):
- an activating member (16) optionally operatively connected to a mouthpiece cap (15) or an activating button said activating member (16) comprising an engagement section (18), said section being arranged for releasable engagement with a wing (13,23,25) of the inner winder (2).

4. Inhalation device according to any of the preceding claims comprising a blister cover (3) having a longitudinal slit (8), said blister cover (3) being arranged coaxially inside said outer shell (1) at a distance allowing for the accommodation of a helically wound blister pack (4), and outside said inner winder (2).

5. Inhalation device according to claims 3 or 4, wherein a hinged cap (15) for covering the mouthpiece (14) is provided, and that the cap (15) is connected to the activating member (16), such that pivoting of the cap (15) engages or disengages the engagement section (18).

6. Inhalation device according to claims 3 or 4, wherein an activating button is provided in the outer shell (1), either as a sliding button or as a depression button, where said button is connected to the activating member (16), such that activation of the button engages or disengages the engagement section (18).

7. Inhalation device according to any of the preceding claims, wherein the device is suitable for substances which may be chosen among fine or coarse powders such as medicaments, candy, or solids such as pills, capsules, candy or liquids such as medicaments, mouth wash, candy and the like.

## Patentansprüche

1. Inhalationsvorrichtung zur Verwendung mit einer Blisterpackung (4), wobei die Blisterpackung (4) eine Basisfolie (9) und eine Deckfolie (10), die lösbar aneinander befestigt sind, umfasst, wobei die Blisterpackung (4) eine Mehrzahl von Behältern (5) zum Aufnehmen einer Substanz aufweist, die entweder in der Basisfolie (9) oder der Deckfolie (10) ausgebildet sind, wobei die Behälter (5) entlang der Länge von der Blisterpackung (4) beabstandet sind; die Inhalationsvorrichtung umfassend: eine äußere Hülle (1), wobei an einem ersten Ende der äußeren Hülle ein Mundstück (14) und ein innerer Wickler (2) mit Mitteln zum Aufnehmen und Halten eines Endes von einer der Folien (9, 10), die die Blisterpackung (4) bilden, vorgesehen sind, **dadurch gekennzeichnet, dass**
- der innere Wickler (2) koaxial innerhalb der äußeren Hülle (1) angeordnet ist und die Vorrichtung zusätzlich umfasst:
- einen durch Atem auslösbaren Auslösemechanismus umfassend Ausrichtmittel zum Drehen des inneren Wicklers (2) um eine vorbestimmte Strecke, wenn die Auslösevorrichtung ausgelöst wird, so dass die eine der Folien, die die Blisterpackung bildet, auf den inneren Wickler gewickelt wird, wodurch die Substanz, die in der Blisterpackung gespeichert ist, freigesetzt wird.

2. Inhalationsvorrichtung nach Anspruch 1, wobei die äußere Hülle (1) einen zylindrischen Aufbau, optional im Wesentlichen kreisförmig oder oval im Querschnitt ist, und an der Innenseite der äußeren Hülle (1) Rillen (6) in der Längsrichtung vorgesehen sind, die im Wesentlichen in der Größe den Blisterbehältern (5) entsprechen, wobei sich die Rillen (6) im Wesentlichen in der Länge der äußeren Hülle (1) erstrecken.

3. Inhalationsvorrichtung nach Anspruch 1, wobei der Auslösemechanismus umfasst:
- eine oder mehr Auskragungen (19 ,23, 25), die einstückig mit dem Umfang des inneren Wicklers (2) vorgesehen sind;
- ein Ausrichtelement (20), das koaxial innerhalb des inneren Wicklers (2) angeordnet ist und das eine im wesentlichen konstante rotatorische Ausrichtkraft auf den inneren Wickler (2) ausübt;
- eine zentrale Achse mit einem Querschnitt, der eine Anzahl von Vorsprüngen (22, 26) umfasst, die an einem Ende der Achse angeordnet sind;
- eine Klappe (21), die um eine Achse senkrecht zu der Längsachse der Vorrichtung schwenkbar ist, umfassend wenigstens einen Vorsprung, der für einen lösbaren Eingriff mit einer Auskragung (19, 23, 25) des inneren Wicklers (2) angeordnet ist, wobei die Klappe angrenzend an das gegenüberliegende Ende des Mundstücks (14) angeordnet ist und wobei die Klappe (21) eine Fläche und Form aufweist, die im Wesentlichen dem inneren Querschnitt der äußeren Hülle (1) entspricht;
- ein Aktivierungselement (16), das optional in Wirkverbindung steht mit einer Mundstückkappe (15) oder einem Aktivierungsknopf, wobei das Aktivierungselement (16) einen Eingriffsabschnitt (18) umfasst, wobei der Abschnitt zum lösbaren Eingriff mit einer Auskragung (19, 23, 25) des inneren Wicklers (2) angeordnet ist.

4. Inhalationsvorrichtung nach einem der vorhergehenden Ansprüche, umfassend eine Blisterabdeckung (3), die einen Längsschlitz (8) aufweist, wobei die Blisterabdeckung (3) koaxial innerhalb der äußeren Hülle (1) in einem Abstand angeordnet ist, der die Unterbringung einer helixartig gewundenen Blisterpackung (4) erlaubt und außerhalb des inneren Wicklers (2) angeordnet ist.

5. Inhalationsvorrichtung nach Anspruch 3 oder 4, wobei eine klappbare Kappe (15) zum Abdecken des Mundstücks (14) vorgesehen ist und die Kappe (15) so mit dem Aktivierungselement (16) verbunden ist, dass ein Schwenken der Kappe (15) den Eingriffsabschnitt (18) in Eingriff bringt oder aus dem Eingriff bringt.

6. Inhalationsvorrichtung nach Anspruch 3 oder 4, wobei ein Aktivierungsknopf an der äußeren Hülle (1) entweder als Schiebeknopf oder als Druckknopf zur Verfügung gestellt wird, wobei der Knopf mit dem Aktivierungselement (16) verbunden ist, so dass die Aktivierung des Knopfes den Eingriffsabschnitt (18) in Eingriff bringt oder aus dem Eingriff bringt.

7. Inhalationsvorrichtung nach einem der vorhergehenden Ansprüche, wobei die Vorrichtung für Substanzen geeignet ist, die aus feinen oder groben Pulvern wie z.B. Medikamenten, Süßigkeiten, oder Feststoffen wie z.B. Tabletten, Kapseln, Süßigkeiten oder Flüssigkeiten wie z.B. Medikamenten, Mundwasser, Süßigkeiten und dergleichen ausgewählt sind.

## Revendications

1. Dispositif d'inhalation destiné à être utilisé avec un emballage coque (4), dans lequel ledit emballage coque (4) comprend une feuille de base (9) et une feuille de couvercle (10) fixées de manière détachable l'une par rapport à l'autre, dans lequel ledit emballage coque (4) a une pluralité de récipients (5) formés dans la feuille de base (9) ou la feuille de couvercle (10) pour contenir une substance, dans lequel les récipients (5) sont espacés le long de la longueur de l'emballage coque (4), le dispositif d'inhalation comprenant une coque externe (1) dans laquelle dans une première extrémité de la coque externe, on trouve un embout buccal (14), et
un enrouleur interne (2) avec des moyens pour recevoir et maintenir une extrémité de l'une des feuilles (9, 10) composant l'emballage coque (4) ;
**caractérisé en ce que** :
l'enrouleur interne (2) est agencé de manière coaxiale à l'intérieur de ladite coque externe (1), et le dispositif comprend en outre :
un mécanisme de déclenchement activé par la respiration comprenant des moyens de sollicitation pour faire tourner l'enrouleur interne (2) sur une distance prédéterminée lorsque ledit mécanisme de déclenchement est déclenché, de sorte que l'une des feuilles composant l'emballage coque est enroulée sur l'enrouleur interne, exposant ainsi la substance stockée dans l'emballage coque.

2. Dispositif d'inhalation selon la revendication 1, dans lequel la coque externe (1) a une configuration cylindrique, facultativement avec une section transversale sensiblement circulaire, elliptique ou ovale, et en ce que sur le côté interne de la coque externe (1), on prévoit des rainures (6) dans la direction longitudinale, correspondant sensiblement du point de vue de la taille aux récipients (5) de l'emballage coque, lesdites rainures (6) s'étendant sensiblement sur la longueur de la coque externe (1).

3. Dispositif d'inhalation selon la revendication 1, dans lequel le mécanisme de déclenchement comprend :
une ou plusieurs ailes (19, 23, 25) prévues de manière solidaire avec la circonférence de l'enrouleur interne (2) ;
un élément de sollicitation (20) agencé de manière coaxiale à l'intérieur de l'enrouleur interne (2) poussant une force de sollicitation rotative sensiblement constante sur l'enrouleur interne (2) ;
un essieu central avec une section transversale comprenant un certain nombre de boutons (22, 26) agencés dans une extrémité dudit essieu ;
un volet (21) pouvant pivoter autour d'un axe perpendiculaire à l'axe longitudinal du dispositif comprenant au moins un bouton qui est agencé pour la mise en prise détachable avec une aile (13, 23, 25) de l'enrouleur interne (2), ledit volet étant agencé de manière adjacente à l'extrémité opposée de l'embout buccal (14), et le volet (21) ayant une surface et une forme correspondant sensiblement à la section transversale interne de la coque externe (1) ;
un élément d'activation (16) raccordé de manière facultativement opérationnelle à un capuchon (15) d'embout buccal ou un bouton d'activation, ledit élément d'actionnement (16) comprenant une section de mise en prise (18), ladite section étant agencée pour la mise en prise détachable avec une aile (13, 23, 25) de l'enrouleur interne (2).

4. Dispositif d'inhalation selon l'une quelconque des revendications précédentes, comprenant un couvercle de coque (3) ayant une fente longitudinale (8), ledit couvercle de coque (3) étant agencé de manière coaxiale à l'intérieur de ladite coque externe (1) à une certaine distance permettant de recevoir un emballage coque (4) enroulé de manière hélicoïdale, et à l'extérieur dudit enrouleur interne (2) :

5. Dispositif d'inhalation selon les revendications 3 ou 4, dans lequel on prévoit un capuchon articulé (15) pour recouvrir l'embout buccal (14), et en ce que le capuchon (15) est raccordé à l'élément d'activation (16) de sorte que le pivotement du capuchon (15) met en prise ou dégage la section de mise en prise (18).

6. Dispositif d'inhalation selon les revendications 3 ou 4, dans lequel on prévoit un bouton d'activation dans la coque externe (1), sous la forme d'un bouton coulissant ou d'un bouton pression, où ledit bouton est raccordé à l'élément d'activation (16), de sorte que l'activation du bouton met en prise ou dégage la section de mise en prise (18).

7. Dispositif d'inhalation selon l'une quelconque des revendications précédentes, dans lequel le dispositif est approprié pour des substances qui peuvent être choisies parmi des poudres fines ou grossières telles que des médicaments, des bonbons, ou des solides comme des pilules, des comprimés, des bonbons ou des liquides tels que des médicaments, des bains de bouche, des bonbons et similaires.
